# EUROPEAN PATENT APPLICATION

(11) **EP 2 641 599 A1**
(43) Date of publication of application: **25.09.2013**
(21) Application number: 13386001.5
(22) Date of filing: 14.01.2013
(51) Int. Cl.: A61K 31/355, A61K 9/48, A61P 35/00, A61P 9/00

(54) **Composition of an oral dosage form of vitamin E esters combination with enhanced antioxidant activity.**

(30) Priority: 17.01.2012 GR 2012100033
(71) Applicant: Papathanasiou, Georgios, 154 52 P.Psychiko Attikis (GR)
(72) Inventor: Papathanasiou, Georgios, 154 52 P.Psychiko Attikis (GR)

(57) **Abstract**

A stable composition of two esters of vitamin E, acetate and acid succinate in proportions ranging from 5% to 95% for each one of them in a oily vehicle into a soft capsule (softgel), which is prepared by the methods either of drop formation or by the method of rotating die rolls. With the drop formation method the solid succinate ester of vitamin E is encapsulated in a softgel utilizing its low melting point (76°-77°). This composition enhances the synergy of the two esters as antioxidants against the free radicals, which are responsible for cardiovascular diseases and for some specific cancers such as those of prostate, throat and colon.

## Description

At first, the present invention refers to the composition of a stable , non-toxic and of improved bioavailability oral dosage form containing a new combination of the fat-soluble antioxidant vitamin E esters¹, α-tocopheryl acetate (α-TA) and α-tocopheryl acid succinate (α- TS), in an oily solvent / vehicle into a soft gelatin capsule (softgel), resulting in the synergistic action of these two vitamin E esters, enhancing, thus, their concomitant antioxidant action against free radicals which are responsible for cardiovascular illnesses ^{2,3} and certain cancers such as prostate cancer ^{4, 5,6,7}, pharynx⁸ and colon⁹ and, secondly, in their preparation process, with the method of drop formation which taking advantage of the low melting point of α-tocopheryl acid succinate encloses this solid substance, in the form of a finely partitioned suspension, in a oily vehicle and thereafter in a soft capsule (softgel), . This combination may also be prepared with the method of rotating die rolls.

Vitamin E has been recognized as a factor with important antioxidant properties². Numerous studies, as it is shown in pages 21, 22 and 23 of this document, have certified the beneficial effect of vitamin E in human health. Specifically, it has been associated with decreasing the risk of cardiovascular diseases, the inhibition of Alzheimer disease progression and it constitutes an important factor in prevention of various cancers, like those of prostate, throat and colon.

Vitamin E is found in nature, in different plants and mainly in wheet grains, soybeans, corn, sunflower seeds etc. The natural vitamin E is a mixture of eight different molecules which is composed of four isomers of tocopherols-α, β, γ and δ and four isomers of tocotrienols - α, β, γ and δ. All eight isomers have a structure of a 6- chromanole ring and an aliphatic side chain with twelve carbon atoms containing two methyl-groups in the middle of the chain and two other methyl-groups at the end, . The side chain of tocopherols is saturated, while the side chain of tocotrienols, which are structurally similar to the tocopherols, has three double bonds at positions 3, 7 and 11. Isomers of both tocopherols and tocotrienols differ in their number and position of substituents - methyl groups on the aromatic chromanolic ring. The α-form has three methyl groups, the β-form and γ-form have two methyl groups each, while the δ-form has only one methyl group. Vitamin E also contains a phenolic hydroxyl group as the active portion of the molecule which binds with free radicals using hydrogen and / or electron exchange rendering the molecule one of the most important known antioxidants factors.

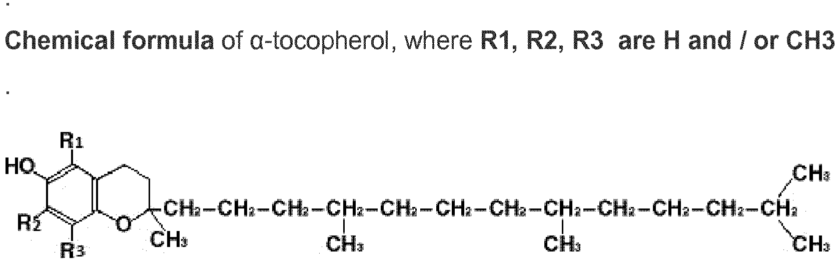

Tocotrienols are more active as antioxidants but their antioxidant effectiveness is greatly reduced because they are poorly absorbed by the gastrointestinal tract, poorly distributed by the blood to the tissues and rapidly metabolized and eliminated from the body.

At room temperature, the isomers of vitamin E are light yellow oils, fairly stable to heat and acids. They degrade under alkaline conditions ultraviolet light and oxygen.

An antioxidant agent is any substance which, after its absorption by the gastrointestinal tract, participates in physiological, biochemical and cellular processes that inactivate free radicals or inhibit chemical reactions triggered by them.

The "free radicals" are unstable ions, mainly oxygen, with high reactivity, which circulate in the blood as a result of endogenous or exogenous processes and effects. A major source of "free radical" production is the metabolism of the human body. Other sources of "free radical" production are the alcohol intake from alcoholic beverages, smoking and environmental factors. These free radicals extract electrons from other molecules of the body to be stabilized and thereby cause oxidative damages. Antioxidants protect the human body from these oxidative damages by providing their electrons to the free radicals, which would have to take otherwise from other healthy molecules of the human body in order to become stable.

Vitamin E belongs to the fat-soluble vitamins and, because of this liposolubizing property, enters and remains in the cell membranes, forming an effective first line of defense of both cell membranes and DNA from oxidative attacks against them. Vitamin E is found in many foods and is absorbed at higher rates by the intestinal tract in the presence of fatty foods. For this reason, it is recommended to be taken during or immediately after meals. Foods rich in vitamin E are vegetables, fruits, many nuts, fish, eggs, edible oils, especially olive oil, whole grains, soybeans, etc. However, because most foods today are submitted to a variety of treatments before arriving at consumption, a large portion of vitamin E contained in them is destroyed. Also, the dietary habits of the modern man, at least the one who lives in Western societies, have changed to the worst (from home made food to fast food with excessive fat content) depriving his body of the precious healthy ingredients, including vitamin E. For these reasons, the additional intake of vitamin E with dietary supplements is necessary so that the human body can secure the protective benefits of vitamin E

Today, vitamin E has been assimilated to α-tocopherol, which is the most active form, and, for this reason, almost all nutritional supplements in the market contain, mainly, α-tocopherol.

Due to the instability of tocopherols at ambient conditions, they are administered in the form of acetate or acid succinate esters which contribute decisively in improving their stability, although they decrease their bioavailability¹¹. Therefore, in order to maintain an effective level of vitamin E in the body, it is necessary to administer higher doses

For more than 50 years, a medicinal product has been produced by the pharmaceutical company, GAP s.a., approved by the National Organization for Medicines (EOF) and is still available in Greece under the trade name EVIOL™ in the form of soft capsules (softgels) with a shelf life of five (5) years. EVIOL™ is produced at the manufacturing facilities of the company with the method of drop formation. Each soft capsule of EVIOL™ (softgel) contains a solution of 100 mg (100 IU) of vitamin E in the form of α-tocopheryl acetate in an oily vehicle, Some of the most beneficial indications of EVIOL™ are:
- Certain anemias and especially hemolytic anemia.
- Intermittent claudication.
- Neuropathy and vasoneurosis.
- Cardiovascular and vascular disease.
- Avitalipoproteinaimia.
- Hypercholesterolemia.
- Delaying the aging process.
- Gerontology.
- Skin diseases, particularly common acne and scleroderma.
- Allergic Rheumatic diseases and diseases of collagen.
- Muscular dystrophy and other muscle diseases.
- Fertility disorders.
- Menopause disorders
- Immaturity of the neonatal lung.
- Contribution to wound healing
- Suspensory effect on myopia development
- Contribution to the prevention of certain cancers.

In combination with vitamin A, protects the lungs from air pollution.

In the scientific literature listed in pages 21 to 23, was found that, according to numerous long-term epidemiological studies, the naturally derived d-α-tocopheryl acid succinate or the synthetic dl-α- tocopheryl acid succinate, are important antioxidant agents which prevent carcinogenesis in certain organs of the body like, prostate, pharynx and colon, while they retain the other beneficial properties of vitamin E, as shown in the indications of EVIOL™.

As a result of the study of the literature, we arrived at the conclusion that it would be an excellent idea to strengthen the already existing and highly successful proprietary medicinal product EVIOL™ by incorporating to it the d-α-tocopheryl succinate, creating, thus, a new original formulation, which would exploit the synergy of the two constituents by multiplying the well known and proven, for more than 50 years, beneficial and therapeutic properties of d-α-tocopheryl acetate with the highly protective properties against certain cancers of the d-α-tocopheryl succinate.

Until today, there has not been a product which utilizes the synergistic action of the two esters of vitamin E and especially in the field of prevention of certain kind of cancers. Moreover, there has not been a combination of these two esters in softgel form that is produced by the method of drop formation, since the α-tocopheryl succinate is insoluble at ambient conditions, a factor which was an obstacle until today, because it did not allow the production of softgels with solid content using the method of the drop formation.

The aim of this invention is the production of a new, standardized, stable, non- toxic oral dosage form with improved bioavailability, by encapsulating combinations of α-tocopheryl acetate and α-tocopheryl succinate in an oily vehicle and in various amounts and proportions using either the method of drop formation or the method of the rotating die rolls. In particular, using an oily vehicle, imroved bioavailability of the α-tocopherol esters which are contained into the softgels is achieved, relieving, this way, the user from taking the dose strictly after having a fat meal.

The new combination of fat-soluble esters of α-tocopherol with their improved bioavailability, facilitates the release of active ingredients in the intestine, resulting in a) in the immediate circulation in the blood of the bound with lipoproteines¹² after hydrolysis and the unbound α-tocopheryl succinate retaining its anticancer properties, (Prasad KN et al Human Press pp 265-288, 1995) and b) the synergistic action of of both derivatives of α-tocopherol after hydrolysis, by enhancing simultaneously their antioxidant properties for the benefit of the human health.

The non-hydrolysed α-tocopheryl acetate acts as mediator on the anti-cancer properties of the α-tocopheryl succinate , and the α-tocopherol which is produced after hydrolysis of both derivatives reacts a) with free radicals reducing thus their destructive effect by creating a α-tocopheroxyl free radical which is much less harmful and is converted to α-tocopherol in the presence of a reducing substance such as vitamin C and b) with the low-density lipoprotein (LDL), which helps to reduce the adhesion of platelets and other lipoproteins in the arteries, reducing thus the probability of heart disease incidences and also helps to prevent any further oxidation of DNA which will cause mutations or cancer. The non-hydrolysed α-tocopheryl succinate acts repressively against the proliferation of cancer cells in various organs of the human body, such as prostate, pharynx and colon^{13, 14,15,16,17,18,19}.

Both esters are stable under normal conditions and remain stable in their dosage form within the soft capsules.

The stability of the product from the beginning of the production until its consumption by the end user, is guaranteed by the strict precautions taken during their preparation processes and their storage, as well.

The advantages of these methods are the (easy) mixing of a oily ester with a solid ester in a oily vehicle and then the encapsulation of the fluid mixture in a soft capsule by the method of the drop formation or the method of rotating die rolls, ensuring, thereby, the co existence and the uniform content of the active substances during both the process and into the end product.

The preparation method of the present combination with the drop formation is innovative in design and implementation, because it combines two esters of different physical states, liquid and solid, in one liquid state, without decomposition products like, tocopheryl hydroquinone, dimer-, trimer- and some other water soluble compounds.

Both methods are advantageous because they easily prepare mixtures of the two esters in varying amounts, from 50mg to 950mg each.

Therefore, the purpose of this invention was to find a way to simultaneously formulate the two esters of vitamin E, which have complimentary actions, in appropriate scientific precalculated doses for maximum absorption, taking into account that, an increased dose of vitamin E does not necessarily mean a corresponding increase in absorption by the human body (Meydani, M., CRC Press, 1996, pp 110-131, Trader, M.G., Williams and Wilkins, 1999, pp 347-362 κατ Leonard, S.W. et al. , American Society for Chemical Nutrition, 2004). Thus, the proposed total daily dose of this new combination of the two esters of vitamin E, the acetate and the acid succinate ester of α-tocopherol, is to be divided into smaller doses, so that the actual absorption of these esters by the human body is maximized. This means, first, that the concentration of these two esters per dosage unit (softgel) must be low e.g. 50-100mg of each ester /softgel, and, secondly, the administration must be repeated for a few times a day, according to each case. In special cases, there will be access to higher doses/administration. That is why, in this present invention are described dosage formulations with higher concentrations of both esters of vitamin E, up to 950mg/softgel each.

In the present invention it is presented a stable oral dosage form prepared by the method of drop formation or by the method of rotating die rolls that contains an original combination of α-tocopheryl acetate (α-TA) and α-tocopheryl succinate (α-TS), exploiting this way, the synergy of these two esters of vitamin E which 1) works not only cumulatively but in a multiple way as well, maximizing the antioxidant / binding / neutralizing properties of vitamin E on free radicals, preventing further the adhesion of platelets and lipoproteins in the arteries thereby, reducing, thus, the mortality due to heart disease and oxidation of DNA that causes mutations or cancer and 2) utilizes the inhibiting and repressive capacity of α-TS against the expansion of cancer on various organs of the human body such as prostate, throat and colon. Further, the product, when it is prepared by the method of drop formation, exploits the very low melting point (76°-77°) of α-TS, achieving, for the first time, the encapsulation of the solid α-tocopheryl succinate (α-TS) in the form of finely partitioned suspension into an oily vehicle , improving this way the bioavailability and the synergy of the two compounds in the combination of this invention.

The patent review showed that the α-tocopheryl succinate (α-TS) is not presented in soft capsules which are prepared by the method of drop formation, in the form of finely partitioned suspension in an oily vehicle, and in combination with the α-tocopheryl acetate (α-TA) on the intension of improving bioavailability and enhance the synergy of these two powerful antioxidant esters of vitamin E. The same new combination may also be prepared by the method of the rotating die rolls.

There are patents for a) aqueous suspension compositions and anhydrous suspensions of lipid soluble vitamins, US Pat. No 3,253,992, b) dermatological and cosmetic applications, US Pat. No 4,551,332, and c) the coexistence of aqueous suspensions with the molten fat-soluble vitamin E in a surfactant agent to restore hydrophilic / lipophilic balance. However, no patent was found reporting the combination of the two esters of vitamin E in an oily vehicle in the form of a softgel, prepared by the drop formation method, which is easy and simple to take orally, aiming at the improvement of the bioavailability and the synergy enhancement of the two esters of the vitamin E.

The purpose of this invention is to present, on a scientific basis, a low cost product with high health value. To understand the need of vitamin E oral intake, it is worth to mention that, in order for the body to take 400IU of vitamin E, an amount which is typically found in a capsule, it should consume 1200g sunflower seeds(6.25Kcal/gr) or 2,4 Kg grain (3.35Kca;/gr) or 972mL corn oi (8.23Kcal/gr) equivalent to 8000 cal a day.. Another reason supporting the oral intake of this new combination of the two esters of vitamin E, is because the bioavailability is improved due to the synergy of these two esters as they are simultaneously released in the intestinal track.

Investigation of the patent literature showed that the procedure of drop formation has not been used in forming a soft capsule (softgel), which will enclose solid α-tocopheryl succinate in the form of finely partitioned suspension in a oily vehicle alone or in combination with α-tocopheryl acetate.

In the present invention it is described a dosage form which takes advantage of the low melting point , 76°-77°, of α- tocopheryl succinate and achieves the encapsulation of this solid form of vitamin E in a soft capsule (softgel) which is manufactured by the method of the droplet formation.

In the present invention is also described, the dosage form which falls within the limits set by the Scientific Committee on food supplements, 300mg of α-tocopherol equivalents per day, (EFSA journal (2008) 640,1-34), as well as the advantages of the synergy of two esters when administered concurrently.

Following the release of the two fat-soluble active esters of α-tocopherol in the intestinal track, part of them, after hydrolysis, will react with lipoproteins, another part of them will circulate in the bloodstream intact, reaching the body organs and will probably act inhibitory or repressively on them, if they are in a state of carcinogenesis and the excess will be eliminated. The new dosage form described in this invention and the method of administration are designed to have maximum desired effect , because, as it is known, any excess of vitamin E is excreted by the human body. In other words, the human body has specific and limited ability to absorb vitamin E, therefore, any excess is useless and thus unabsorbed by the human body.

Another crucial factor in this dosage formulation is the presence of acetate ester (α-TA) of α- tocopherol, which mediates for the best action of acid succinate ester (α-TS) of α- tocopherol, when there is a state requiring inhibition or repression for cancer, (KN Prasad, Review). The combination of the two esters of α- tocopherol uses for the first time the property of, suppression, prevention or inhibition of proliferation of certain cancers, including prostate cancers, throat and colon, of α-TS along with its antioxidant property.

As oily vehicles, vegetable oils may be used , alone or mixtures thereof in various proportions between them ranging from 5% to 95% each. Oils, for example, but not exclusively" like soy bean oil, sunflower oil, sesame oil, etc. may be used.

The two esters of α- tocopherol are dissolved or dispersed in the oily vehicle at various proportions among them ranging from 5% to 95% for each one of them. Each softgel capsule may contain 50 mg to 950mg from each ester.

The shell of the softgels may consist of gelatin, hydrated or anhydrous glycerin, hydrated or anhydrous sorbitol or a mixture of glycerin - sorbitol or a mixture of sorbitol-sorbitan(s)-polyols and coloring agents, flavoring agents and preservatives, as well.

All components of the composition of the present invention should be approved for use in drugs and/or foods.

The preparation method of the new composition of the dosage form of Vatamin E esters includes the following steps:
(a) Suspension of the solid α-tocopheryl acid succinate in the oily vehicle which can be soybean oil, sunflower oil, sesame oil or other vegetable oil under stirring and nitrogen.
(b) Heating the suspension of α-tocopheryl acid succinate in the oily vehicle up to 77°, under constant stirring and always under nitrogen.
(c) Leave the suspension of α-tocopheryl acid succinate in the oily vehicle at 77°, under constant stirring and always under nitrogen until the solution becomes clear.
(d) Slow cooling of the suspension of α-tocopheryl acid succinate in the oily vehicle up to 40°. under constant stirring and always under nitrogen.
(e) Addition of α-tocopheryl acetate.under stirring and always under nitrogen.
(f) Leave the mixture at 40° under stirring and always under nitrogen for 30 min.
(g) Deaerate the mixture maintaining the temperature at 40°.
(h) After deaeration, maintain the mixture under vacuum until it is transferred to one of the two encapsulation machines (drop formation or rotating die rolls).
(i) Dispersion of gelatin in granular form in a mixture of glycerol - water at ambient temperature.
(j) In the above mixture of gelatin" if necessary or desired, coloring and/or flavoring agents and / or preservatives may be added.
(k) The mixture of gelatin is heated up to 65° or 85° ,depending on the method, under stirring and vacuum until the gelatin solution becomes clear.
(l) The clear gelatin solution is deaerated and maintained at 60° until it will be used and during the whole softgels production process.
(m) The mixture of the esters of α-tocopherol is transferred to the encapsulation machines where they are constantly kept under nitrogen throughout the whole production process, depending on the method to be used.
(n) Softgels production that contain combinations of α-tocopheryl succinate κατ α-tocopheryl acetate.

### Example 1

For the preparation of 50000 softgels, of the composition of the present invention with the method of drop formation, where each softgel contains 50 mg of α-tocopheryl acid succinate and 100 mg of α-tocopheryl acetate ,the following are required:

### A-Fill

| | |
|---|---|
| α-tocopheryl acid succinate | 2500 g |
| α-tocopheryl acetate | 5000 g |
| Sesame Oil | 5625 g |

### B.-Shell

| | |
|---|---|
| Gelatin | 3150,00 g |
| Glycerin 99,5% | 1050,00 g |
| D.I. Water | 6000,00 g |
| Patent Blue V (E131) | 0,04 g |
| Ponceau 4R (E110) | 2,00 g |

### C.-Method

(1) Suspension of the solid α-tocopheryl acid succinate in sesame oil under stirring and nitrogen atmosphere.
(2) Heating the suspension of α-tocopheryl acid succinate in sesame oil up to 77°, under constant stirring and always under nitrogen atmosphere.
(3) Leave the suspension of α-tocopheryl acid succinate in sesame oil at 77°, under constant stirring and always under nitrogen until the solution becomes clear.
(4) Slow cooling of the suspension of α-tocopheryl acid succinate in sesame oil up to 40°, under constant stirring and always under nitrogen.
(5) Addition of α-tocopheryl acetate under stirring and always under nitrogen.
(6) Leave the mixture at 40° under constant stirring and always under nitrogen for 30 min.
(7) Deaerate the mixture maintaining the temperature at 40°.
(8) After deaeration, maintain the mixture under vacuum until it is transferred to the encapsulation machine , GLOBEX Mk II.
(9) Disperse gelatin in granular form in the mixture of glycerol - water in which the two coloring agents had previously dissolved at room temperature.
(10) The mixture of gelatin is heated up to 60° under slow stirring until the gelatin solution becomes clear.
(11) The clear gelatin solution is kept at 60° to mature until the following morning, at which time it can be used.
(12) The gelatin solution is placed in the special container of the encapsulation machine with the method of he droplet formation, GLOBEX Mk II and is heated at 60° where it is maintained throughout the whole production process.
(13) The deaerated mixture of vitamins is placed in the special container of the encapsulation machine with the droplet formation method, GLOBEX Mk II and it is heated at 40° under nitrogen , where it is maintained throughout the whole production process.
(14) The GLOBEX Mk II encapsulation machine is adjusted to produce softgels with fill weight 262,5 mg/cap.
(15) The produced softgels are placed in a special drying oven, where they are kept for 72 hours at 20°-22° and at relative humidity, RH up to 40% so that the production will be completed.

### Example 2

For the preparation of 40000 softgels, of the composition of the present invention with the method of droplet formation, where each softgel contains 100 mg of α-tocopheryl acid succinate and 100 mg of α-tocopheryl acetate, are required:

### A-Fill

| | |
|---|---|
| α-tocopheryl acid succinate | 4000 g |
| α-tocopheryl acetate | 4000 g |
| Sunflower Oil | 6000 g |

### B.-Shell

| | |
|---|---|
| Gelatin | 3150,00 g |
| Glycerin | 1050,00 g |
| D.I. Water | 6000,00 g |
| Patent Blue V (E131) | 0,77 g |
| Sunset Yellow FCF (E110) | 1,15 g |

### C.-Method

(1) Suspension of the solid α-tocopheryl acid succinate in sesame oil under stirring and nitrogen.
(2) Heating the suspension of α-tocopheryl acid succinate in sesame oil up to 77°, under constant stirring and always under nitrogen.
(3) Leave the suspension of α-tocopheryl acid succinate in sesame oil at 77°, under constant stirring and always under nitrogen atmosphere until the solution becomes clear.
(4) Slow cooling of the suspension of α-tocopheryl acid succinate in sesame oil up to 40°. under constant stirring and always under nitrogen atmosphere.
(5) Addition of α-tocopheryl acetate under stirring and always under nitrogen atmosphere.
6) Leave the mixture at 40° under constant stirring and nitrogen atmosphere for 30 min.
7) Deaerate the mixture, maintaining the temperature at 40°.
8) After deaeration, maintain the mixture under vacuum until it is transferred to the encapsulation machine, GLOBEX Mk II.
9) Disperse gelatin in granular form in the mixture of glycerol - water in which the coloring agents had previously been dissolved at room temperature.
10) The mixture of gelatin is heated up to 60° with stirring until the gelatin solution becomes clear..
11) The clear gelatin solution is kept at 60° to mature until the next day, at which time it can be used.
12) The gelatin solution is placed in the special container of the ecapsulation machine of the method of the drop formation, GLOBEX Mk II and heated at 60° where it is maintained throughout the whole production process.
13) The deaerated mixture of vitamins is placed in the special container of the encapsulation machine of the method of drop formation, GLOBEX Mk II and heated at 40° under nitrogen atmosphere which is maintained throughout the whole production process.
14) The GLOBEX Mk II is adjusted to produce softgels with weight content 350 mg/cap.
15) The produced softgels are placed in a special drying oven, where they remain for 72 hours at 20°-22° and at relative humidity, RH, up to 40% so that the production will be completed.

### Example 3

For the preparation of 200000 softgels, of the composition of the present invention with the method of rotating die rolls, where each softgel contains 100 mg of α-tocopheryl acid succinate and 100 mg of α-tocopheryl acetate, are required:

### A-Fill

| | |
|---|---|
| α-tocopheryl acid succinate | 20000 g |
| α-tocopheryl acetate | 20000 g |
| Soybean Oil | 30000 g |

### B.-Shell

| | |
|---|---|
| Gelatin | 43200,00 g |
| Glycerin | 18150,00 g |
| D.I. Water | 38650,00 g |
| Patent Blue V (E131) | 20,00 g |

### C.-Method

(1) Suspension of the solid α-tocopheryl acid succinate in soybean oil under stirring and nitrogen.
(2) Heating the suspension of α-tocopheryl acid succinate in soybean oil up to 77°, under constant stirring and always under nitrogen atmosphere.
(3)Leave the suspension of α-tocopheryl acid succinate in soybean oil at 77°, under constant stirring and always under nitrogen atmosphere until the solution becomes clear.
(4) Slow cooling of the suspension of α-tocopheryl acid succinate in soybean oil up to 40°. under constant stirring and always under nitrogen atmosphere.
(5) Addition of α-tocopheryl acetate under stirring and always under nitrogen atmosphere.
6) Leave the mixture under constant stirring and nitrogen atmosphere for 30 min.
7) The resulting suspension passes through a colloid mill, which is adjusted to the minimum allowed space between rotor and stator.
8) Deaerate the mixture under vacuum.
9) After deaeration, maintain the mixture under vacuum until it is transferred to the encapsulation machine with the method of the rotating die rolls, TAE SUNG TS-SG 10.
10) Disperse gelatin in granular form in the mixture of glycerol - water, in which the coloring agent had previously been dissolved at room temperature.
11) The gelatin mixture is heated up to 85° under slow stirring and gradual vacuum application so that, the gelatin solution will be deaerated.
12) The vacuum is continuously applied until the gelatin solution becomes clear.
13) The clear gelatin solution is kept at 60° to mature until the next day, at which it can be used.
14) The encapsulation machine TAE SUNG TS-SG 10 is equipped with the appropriate set of die rolls OVAL 6.
15) The tanks of gelatin and suspension of vitamins are connected to the corresponding feeding systems of the machine and the production of softgels begins with weight content 350 mg / softgel.
16) The resulting softgels undergo drying in two steps: a) preliminary, with the method of tumbling and b) with the main process, by placing them into a drying chamber and allow them to stay at 20°-22° and relative humidity, RH 20%, until their production will be completed

### Example 4

For the preparation of 66666 softgels, of the composition of the present invention with the method of rotating molds, where each softgel contains 300 mg of a α-tocopheryl acid succinate and 300 mg of, α-tocopheryl acetate, are required:

### A-Fill

| | |
|---|---|
| α-tocopheryl acid succinate | 20000 g |
| α-tocopheryl acetate | 20000 g |
| Soybean Oil | 30000 g |

### B.-Shell

| | |
|---|---|
| Gelatin | 43200,00 g |
| Glycerin 99.5% | 18150,00 g |
| D.I. Water | 38650,00 g |

### C.-Method

(1) Suspension of the solid α-tocopheryl acid succinate in soybean oil under stirring and nitrogen.
(2) Heating the suspension of α-tocopheryl acid succinate in soybean oil up to 77°, under constant stirring and always under nitrogen atmosphere.
(3) Leave the suspension of α-tocopheryl acid succinate in soybean oil at 77°, under constant stirring and always under nitrogen atmosphere until the solution becomes clear.
(4) Slow cooling of the suspension of α-tocopheryl acid succinate in soybean oil up to 40°. under constant stirring and always under nitrogen atmosphere.
(5) Addition of α-tocopheryl acetate under stirring and always under nitrogen atmosphere.
6) Leave the mixture under constant stirring and nitrogen atmosphere for 30 min.
7) The resulting suspension passes through a colloid mill, which is adjusted to the minimum allowed space between rotor and stator.
8)Deaerate the mixture under vacuum.
9) After deaeration, maintain the mixture under vacuum until it is transferred to the encapsulation machine of the method of the rotating die rolls, TAE SUNG TS-SG 10.
10) Disperse gelatin in granular form in the mixture of glycerol - water, in which the coloring agent had previously dissolved at room temperature.
11) The gelatin mixture is heated up to 85° under slow stirring and gradual vacuum application so that, the gelatin solution will be deaerated.
12) The vacuum is continuously applied until the gelatin solution becomes clear.
13) The clear gelatin solution is held at 60° to mature until the next day, at which time it can be used.
14) The encapsulation machine, TAE SUNG TS-SG 10, is equipped with the appropriate set of cylindrical die rolls OBLONG 20.
15) The containers of gelatin and suspension of vitamins are connected to the corresponding feeding systems of the encapsulation machine and the production of softgels begins with weight content 350 mg / softgel.
16) The resulting softgels undergo ddrying in two steps: a) preliminary with the method of tumbling and b) with the main process, by placing them in a drying chamber and allow them to stay at 20°-22° and relative humidity at RH 20%, until their production will be complete
   **1. The Role of Vitamin E in the** Emerging Field of Neutraceuticals, p. 27-46. Book by Klaus Kramer, Peter Paul Hope, Lester Packer. Nutraceuticals in Health and Disease Prevention
   **2.** Vitamin E and vitamin C supplement use and risk of all-cause and coronary heart disease mortality in older persons: the Established Populations for Epidemiologic Studies of the Elderly. K. G. Losonczy et al. Am Clin Nutr 1996, 64: 190-196.
   **3.** Randomizsed controlled trial of Vitamin E in patients with coronary disease: Cambridge Heart Antioxidant Study (CHAOS). N.G. Stephens et al. Lancet Circulate Vol 347 March 23 1996 781-784.
   **4.** {alpha}-Tocopheryl Succinate Induces Apoptosis in Prostate Cancer Cells in Part through Inhibition of Bcl-xL/Bcl-2 Function. C.-W. Shiau, J.-W. Huang, D.-S. Wang, J.-R. Weng, C.-C. Yang, C.-H. Lin, C. Li, and C.-S. Chen. J. Biol. Chem., April 28, 2006; 281 (17): 11819 - 11825.
   **5.** RRR-{alpha}-Vitamin E Succinate Potentiates the Antitumor Effect of Calcitriol in Prostate Cancer without Overt Side Effects Y. Yin, J. Ni, M. Chen, Y. Guo, and S. Yeh. Clin. Cancer Res., January 1, 2009; 15(1): 190 - 200.
   **6.** Prostate Cancer and Supplamentation with a- Tocopherol and β- Carotene: Incidence and Mortality in a Control Trial. Oili P Heinomen, Demetrius Albanes, Jarmo Vitramo, Philip Taylor.. Journal of the National Cancer Institute, Vol. 90. No. 6 March 18 1998.
   **7.** Vitamin E succinate suppresses prostate tumor growth by inducing apoptosis. Mokenge P. Malafa1*, Frida D. Fokum2, Jennifer Andoh2, Leslie T. Neitzel2, Sucharita Bandyopadhyay3, Rui Zhan3, Megumi liizumi3, Eiji Furuta3, Elizabeth Horvath1 and Kounosuke Watabe3. Int. J. Cancer: 118, 2441-2447 (2006)' 2005 Wiley-Liss, Inc
   **8.** Vitamin Supplement Use and Reduced Risk of Oral and Pharyngeal Cancer. Gloria Gridley1, Joseph K. McLaughlin1, Gladys Block,William J Blot" Maria Glutch and Joseph F Fraumeni jr. American Journal of Epidemiology 1991: Volume 135, Issue 10pp 1083-1092.
   **9.** Relationship between Vitamin and Calcium Supplement Use and Colon Cancer. E. White et al. Cancer Epidemiology, Biomarkers & Prevention. 1997: Vol. 6, 760-774.
   **10.** Multiple Dietary Antioxidants Enhance the Efficacy of Standard and Experimental Cancer Therapies and Decrease Their Toxicity. K. N. Prasad. Integr. Cancer Ther., December 1, 2004; 3(4): 310 - 322.
   **11.** RRR-α-Tocopheryl succinate is a less bioavailable source of vitamin E than all-rac-α-tocopheryl acetate for red drum, Sciaenops ocellatus. Peng Li Xiaoxue Wang, Delbert M. Gatlin III Aguaculture Vol 280, Issues 1-4, 1 August 2008, Pages 165-169
   **12.** Modification of human low density lipoprotein by lipid peroxidation. Esterbauer H, Jurgens G, Quehenberger O. Basic Life Scince 49: 369-373, 1996.
   **13.** a- Tocopheryl Succinate, the Most Effective Form of Vitamine E for Adjuvant Cancer Treatment: A Review. K.N. Prasad, PhD, et al. Journal of the American College of Nutrition Vol. 22, No. 108- 117 (2003).
   **14.** Multiple Dietary Antioxidants Enhance the Efficacy of Standard and Experimental Cancer Therapies and Decrease Their Toxicity. K. N. Prasad. Integr. Cancer Ther., December 1, 2004; 3(4): 310 - 322.
   **15.** Up-regulation of c-Jun-NH2-kinase pathway contributes to the induction of mitochondria-mediated apoptosis by {alpha}-tocopheryl succinate in human prostate cancer cells. K. Zu, L. Hawthorn, and C. Ip. Mol. Cancer Ther., January 1, 2005; 4(1): 43 - 50.
   **16.** Induction of cancer cell apoptosis by alpha-tocopheryl succinate: molecular pathways and structural requirements. Neuzil J, Weber T, Schroder A, et al. FASEB J. 2001;15(2):403-415.
   **17.** Vitamin E and Cancer prevention : methodological aspects Knekt P. In: Ohigshi H,et al. Foods Factors for Cancer prevention. New York:Springen 1997***.***
   **18.** RRR-{alpha}-Tocopherol succinate d o w n-regulates oncogenic Ras signaling. S. Donapaty, S. Louis, E. Horvath, J. Kun, S. M. Sebti, and M. P. Malafa Mol. Cancer Ther., February 1, 2006; 5(2): 309 - 316.
   **19.** Vitamin E succinate is a potent novel antineoplastic agent with high selectivity and cooperativity with tumor necrosis factor-related apoptosis-inducing ligand (Apo2 ligand) in vivo. Weber T, Lu M, Andera L, et al. Clin Cancer Res. 2002;8(3):863-869.
   20. RRR-alpha-tocopheryl succinate-induced apoptosis of human breast cancer cells involves Bax translocation to mitochondria. Yu W, Sanders BG, Kline K. Cancer Res. 2003;63(10):2483-2491.

## Claims

1. A composition of an oral dosage form combining two (2) vitamin E(α-tocopherol) esters, the acetate (α-TA) and the succinate (α-TS), with enhanced antioxidant activity, acting by inactivation of the free radicals generated by the human body which are responsible for cardiovascular diseases and several cancer species such as those of prostate, pharynx and colon.

2. The composition of claim 1 which is presented as a soft gelatin capsule (softgel, soft elastic capsule, SEC).

3. The composition of claims 1 and 2 which contains quantities of the two vitamin E esters ranging between 50 mg and 950 mg each.

4. The composition of claims 1, 2 and 3 where the vehicle of the two vitamin E esters is a vegetable oil such as, for example but not exclusively, sesame oil, sunflower oil, soy bean oil or mixtures thereof in any proportion.

5. The composition of of claims 1, 2, 3 and 4 where the shell of the softgels may consist of gelatin, hydrated or anhydrous glycerin, hydrated or anhydrous sorbitol or a mixture of glycerin - sorbitol or a mixture of sorbitol-sorbitan(s)-polyols and coloring agents, flavoring agents and preservatives, as well.
